## Europäisches Patentamt

## European Patent Office

## Office européen des brevets

(19)

(11) Numéro de publication: **0 157 736**
**B1**

ι

(12) # FASCICULE DE BREVET EUROPÉEN

(45) Date de publication du fascicule du brevet:
**13.01.88**

(51) Int. Cl.⁴: **C 01 F 7/76**, A 61 K 7/38

(21) Numéro de dépôt: **85810107.4**

(22) Date de dépôt: **12.03.85**

(54) **Pierre d'alun de potasse, procédé d'obtention et utilisation d'une telle pierre.**

(30) Priorité: **27.03.84 CH 1536/84**

(43) Date de publication de la demande:
**09.10.85 Bulletin 85/41**

(45) Mention de la délivrance du brevet:
**13.01.88 Bulletin 88/2**

(84) Etats contractants désignés:
**DE FR IT**

(56) Documents cité:
**PATENT ABSTRACTS OF JAPAN, vol. 8, no. 126, 13 juin 1984, page (C-228)(1563); & JP-A-59-36607 (RIKIICHI ENDOU) 28-02-1984**
**PATENT ABSTRACTS OF JAPAN, vol. 7, no. 143, 22 juin 1983, page (C-172) (1288); & JP-A-58-57313 (MORITOMO WAKAMATSU) 05-04-1983**
**Prof. Dr. H. Römpp, "Chemie Lexikon", 6. Aufl., 1966, Franck'sche Verlagshandlung, Stuttgart, p. 5337**
**"Handbuch der Kosmetika und Riechstoffe", I. Band: Die kosmetischen Grundstoffe, H. Janistyn, 3. Aufl., 1978, Dr.A. Hüthig Verlag, Heidelberg, p. 70, 71**

(73) Titulaire: **Verdan, Francis, Les Isles 4, CH- 2015 Areuse/NE (CH)**

(72) Inventeur: **Verdan, Francis, Les Isles 4, CH- 2015 Areuse/NE (CH)**

(74) Mandataire: **North, Mathieu, Place des Halles 13, CH- 2001 Neuchâtel (CH)**

LIBER, STOCKHOLM 1988

## Description

La présente invention concerne un procédé d'obtention d'une pierre d'alun de potasse d'une forme particulière.

Les aluns sont obtenus par mélange, en proportions déterminées, de solutions chaudes de sulfate d'alumine et de sulfate de potassium ou d'ammonium. Par refroidissement, les aluns cristallisent en masse. Les aluns, sous l'action de la chaleur, fondent dans leur eau de cristallisation.

Les applications des aluns sont nombreuses dans des domaines très variés. En particulier, ils sont utilisés en médecine en en pharmacie pour leurs propriétés astringentes et antiseptiques comme gargarismes, dentifrices, antisueur, etc.

Pour leur utilisation dans ces différents domaines, les aluns sont présentés en dissolution dans un liquide, ou en poudre, ou encore en bloc cristallisé.

La présente invention a trait exclusivement à l'alun de potasse cristallisé en masse.

Pour sa commercialisation et son utilisation effective l'alun de potasse cristallisé en masse est concassé en morceaux de 0,02 à 5 kg. Pour les soins corporels externes, l'alun de potasse est le plus souvent présenté sous forme de petits blocs parallélépipédiques ou cubiques appelés "pierres d'alun" dont le poids varie entre 50 et 300 grammes selon les dimensions données.

Ces petits blocs d'alun sont obtenus par sciage à partir d'un gros bloc d'alun cristallisé en masse dont le poids varie entre 500 et 5000 kg. Ensuite, un rognage ou meulage des arêtes et des angles leur donne la forme définitive exempte d'angles vifs pouvant blesser la peau.

Comme une pierre d'alun parallélépipédique a douze arrêtes et huit angles, l'opération de rognage prend nécessairement beaucoup de temps et augmente fortement le prix de fabrication qui peut représenter jusqu'à 90 % du prix de revient du produit terminé.

Utilisées comme désodorisant d'autre part, les pierres d'alun sont principalement destinées à être frottées sous les aisselles qui présentent des formes arrondies. L'allure parallélépipédique généralement donnée aux pierres s'adapte donc mal à l'anatomie de cette partie du corps.

L'objet de l'invention est de fournir un procédé de fabrication économique et rationnel d'une pierre d'alun qui ait une forme bien adaptée pour être frottée sous les aisselles; le procédé est notamment destiné à la production de petits blocs d'alun d'un poids de 0,05 à 5 kg.

Le procédé de fabrication, décrit dans la revendication 1, vise à obtenir une pierre d'alun principalement remarquable en ce que sa forme générale est celle d'un cylindre. Dans une forme d'exécution préférée, la pierre a la forme générale d'un cylindre de révolution. Selon cette forme d'exécution, la pierre a une forme de disque. De préférence, les angles de ce disque sont rognés.

Diverses formes d'exécution du procédé sont décrites dans les revendications 2 à 5.

La forme de la pierre est particulièrement bien adaptée à son utilisation comme désodorisant.

Les dessins illustrent, à titre d'exemples, un produit obtenu selon le procédé, ainsi qu'un procédé destiné à sa fabrication. Sur ce dessin:
- La fi . 1 représente une pierre de forme parallélépipédique conventionnelle, obtenue par sciage selon un art antérieur connu;
- La fig. 2 donne un exemple de mise en oeuvre du procédé de fabrication selon l'invention pour l'obtention d'une pierre d'alun;
- La fig. 3 représente une pierre d'alun de forme cylindrique obtenue par le procédé selon l'invention.

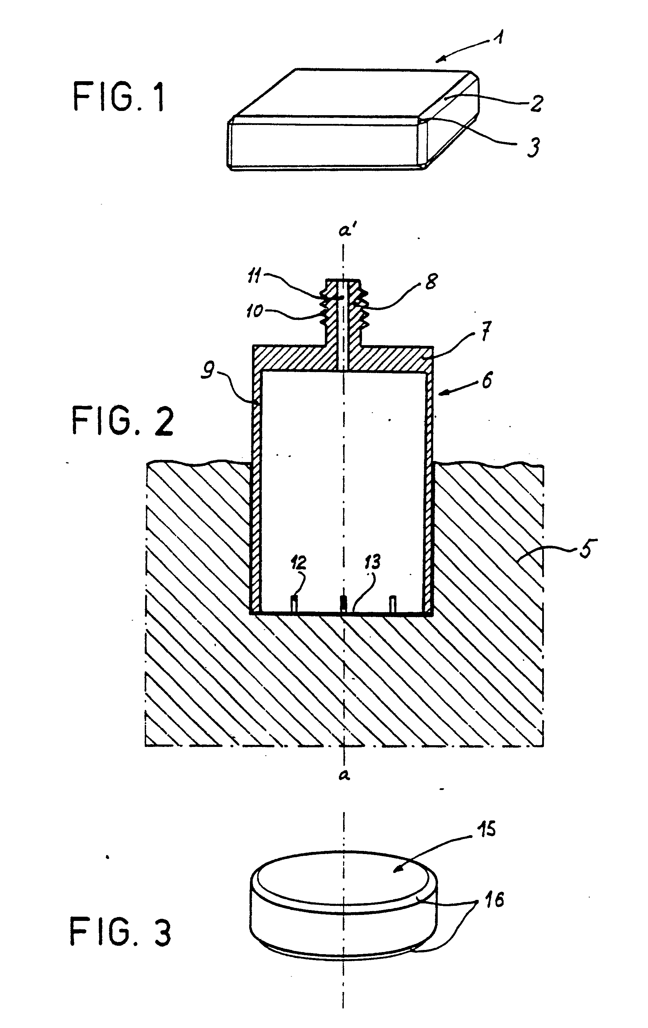

La fig. 1 montre une pierre d'alun conventionnelle 1. Cette pierre est obtenue à partir d'un bloc d'alun par découpage à l'aide d'une scie à ruban, selon une technique connue de l'art antérieur. La forme de la pierre ainsi obtenue est préférentiellement parallélépipédique, car elle correspond à l'utilisation la plus rationnelle de ce procédé de fabrication. Les arêtes 2 et les angles 3 de la pierre 1 sont ensuite rognés, par exemple par meulage, afin d'éviter les blessures ou l'inconfort qui peuvent résulter du contact avec la peau des aspérités présentant un angle vif.

La forme parallélépipédique de la pierre est liée à son procédé de fabrication. Le rognage des douze arêtes et des huit angles associés à cette forme nécessite une opération complémentaire longue et coûteuse. Cette opération peut grandement être simplifiée à l'aide du procédé de fabrication selon l'invention.

Une phase essentielle du procédé proposé est représentée sur la fig. 2. Elle consiste à découper dans un bloc d'alun 5 une carotte non représentée à l'aide d'un outil, lequel, dans cet exemple, est un foret creux 6, par une technique connue en soi dans d'autres industries. Ce foret comprend une base 7 en forme de disque portant, d'un côté, un moyen de fixation 8 et, de l'autre côté, un cylindre creux 9 ayant un axe de symétrie aa' qui est également un axe de symétrie du foret 6. Le moyen de fixation 8 peut être pourvu, par exemple, d'un filetage 10 permettant de fixer le foret sur une machine rotative non représentée pour imprimer au foret un mouvement de rotation autour de l'axe aa'.

Sur la fig. 2 le foret 6 est représenté dans une position où il a déjà pénétré profondément dans le bloc 5. La partie du bloc 5 se trouvant à l'intérieur du cylindre 9 constitue la carotte dans laquelle seront découpées les pierres et qui, dans cette figure, a été enlevée pour montrer la forme de l'extrémité libre du cylindre 9, opposée au disque de base 7. Le pourtour de cette extrémité du cylindre 9 est une couronne diamantée apte à entamer le bloc d'alun. Des fentes 12 sont pratiquées, délimitant des dents 13.

Si le foret 6 est mis en rotation et qu'une force l'appuie contre le bloc 5, il pénétrera dans ce bloc grâce à la couronne diamantée qui enlèvera de la matière. Cet usinage peut être facilité et accéléré en faisant travailler le foret en présence d'un liquide, par exemple de l'eau, qui permet de refroidir l'outil et d'éviter ainsi la fonte de l'alun sous l'effet de la chaleur, et d'évacuer la matière enlevée. Il est avantageux d'utiliser de l'eau sous pression arrivant à l'intérieur du foret 6, comme cela est montré à la fig. 2, par un canal axial 11 traversant la base 7 et le moyen de fixation 8. L'eau s'échappe par les fentes 12, emportant avec elle la matière enlevée.

Une fois que le foret 6 a entièrement pénétré dans le bloc 5, l'usinage de la carotte est terminé.

La carotte ainsi obtenue a la forme d'un cylindre de révolution. Il est possible de l'utiliser telle quelle, sous forme de bâtonnet. Il est toutefois préférable de découper cette carotte en tranches parallèles, par exemple planes et perpendiculaires aux génératrices du cylindre, par des moyens connus, tel que le sciage par une scie à ruban, pour obtenir des pierres de forme pratique. L'opération de sciage, qui se fait pour six faces selon la technique traditionnelle, se réduit ici à une seule opération. D'autre part, les pierres ainsi obtenues ont alors la forme de rondelles ayant seulement deux arêtes à l'exclusion de tout autre angle. L'opération du rognage des autres angles est ainsi supprimée. Quant au rognage des arêtes, l'opération est largement simplifiée, puisqu'elle affecte seulement deux arêtes au lieu de douze. La plus grande partie du prix de revient d'une pierre d'alun obtenue par sciage selon la technique traditionnelle résidant dans son extraction et sa finition, la presente invention apporte un avantage de poids.

La pierre représentée à la fig. 3 présente la forme d'une rondelle cylindrique de révolution dont les arêtes 16 ont été rognées afin d'enlever tous les angles vifs pouvant blesser la peau. La forme arrondie de la pierre s'adapte bien à l'anatomie du creux de l'aisselle, facilitant ainsi son utilisation comme désodorisant corporel.

**Revendications**

1. Procédé de fabrication d'une pierre d'alun de potasse à partir d'un bloc d'alun, caractérisé en ce qu'il consiste à extraire dudit bloc une carotte cylindrique.

2. Procédé de fabrication selon la revendication 1, caractérisé en ce que ladite carotte est débitée en tranches, chaque tranche constituant une pierre, les angles que font les faces tranchées avec la paroi cylindrique étant rognés.

3. Procédé de fabrication selon la revandication 1, caractérisé en ce que ladite carotte est découpée dans ledit bloc à l'aide d'un foret ayant la forme d'un cylindre de révolution creux.

4. Procédé de fabrication selon la revendication 3, caractérisé en ce que le foret présente une couronne diamantée.

5. Procédé de fabrication selon la revendication 3, caractérisé en ce que le découpage de la carotte se fait en présence d'un liquide, ledit liquide étant injecté sous pression dans ledit foret par une ouverture axiale.

**Patentansprïche**

1. Verfahren zur Herstellung eines Kalialaunsteines ausgehend von einem Alaunblock, dadurch gekennzeichnet, dass es darin besteht, dass aus besagtem Block ein zylindrischer Stift herausgetrennt wird.

2. Verfahren gemäss Patentanspruch 1, dadurch gekennzeichnet, dass besagter zylindrischer Stift in Stücke zerteilt wird, welche Stücke jeweils einen Stein darstellen, dessen Kanten zwischen den Schnittflächen und der zylindrischen Wandung abgerundet sind.

3. Verfahren gemäss Patentanspruch 1, dadurch gekennzeichnet, dass besagter zylindrischer Stift aus besagtem Block mittels eines Bohrers, der die Form eines hohlen Rotationszylinders aufweist, herausgeschnitten wird.

4. Verfahren gemäss Patentanspruch 3, dadurch gekennzeichnet, dass der Bohrer einen diamantenbesetzten Schneidkranz aufweist.

5. Verfahren gemäss Patentanspruch 3, dadurch gekennzeichnet, dass das Heraustrennen des zylindrischen Stiftes in Gegenwart einer Flüssigkeit geschieht, welche Flüssigkeit unter Druck durch eine axiale Öffnung in den Bohrer eingespritzt wird.

**Claims**

1. Process for the manufacture of a potash alum stone from a block of alum, characterized in that it consists of extracting a cylindrical core from the block.

2. Process according to claim 1, characterized in that the core is cut up in slices, each slice is a stone, the angles formed by the cut surfaces and the cylinder wall are rounded.

3. Process according to claim 1, characterized in that the core is cut out of the block using a core drill which has the form of a hollow cylinder generated by revolution.

4. Process according to claim 3, characterized in that the core drill is a diamond crown core bit.

5. Process according to claim 3, characterized in that the core drilling takes place in liquid, the liquid being injected under pressure through an axial opening into the core drill.

FIG. 1

FIG. 2

FIG. 3